# EUROPÄISCHE PATENTSCHRIFT

(11) **EP 3 226 950 B1**
(45) Veröffentlichungstag und Bekanntmachung des Hinweises auf die Patenterteilung: **28.08.2019**
(21) Anmeldenummer: 15808343.6
(22) Anmeldetag: 17.11.2015
(51) Int. Cl.: A61M 16/00, A61M 16/01, A61M 16/10, A61M 16/08, A61M 16/22

(54) **ATEMLUFTVERSORGUNG MIT RÜCKATEMSYSTEM**
BREATHING AIR SUPPLY WITH REBREATHING SYSTEM
ALIMENTATION EN AIR RESPIRATOIRE ÉQUIPÉE D'UN SYSTÈME DE RÉINSPIRATION

(30) Priorität: 01.12.2014 DE 102014017712
(43) Veröffentlichungstag der Anmeldung: 11.10.2017
(73) Patentinhaber: Drägerwerk AG & Co. KGaA, 23558 Lübeck (DE)
(72) Erfinder: HEESCH, Ralf, 23568 Lübeck (DE)
(86) Internationale Anmeldenummer: PCT/EP2015/002293
(87) Internationale Veröffentlichungsnummer: WO 2016/087020

(56) Entgegenhaltungen:
- EP-A1- 2 374 509
- WO-A1-99/33523
- DE-A1- 10 047 137
- US-A1- 2002 148 471
- US-A1- 2007 051 367
- US-A1- 2010 199 993

## Beschreibung

Die Erfindung betrifft eine Vorrichtung zur Atemluftversorgung eines Menschen mit einem im Luftversorgungskreislauf angeordneten Rückatemsystem, das mittels eines CO₂-Absorbers wenigstens teilweise in der Ausatemluft des Menschen befindliches CO₂ entfernt und die Luft derart aufbereitet, insbesondere mit Sauerstoff anreichert, dass die so aufbereitete Luft dem Menschen wieder als Einatemluft zuführbar ist.

Vorrichtungen zur Atemluftversorgung eines Menschen mit Rückatemsystem werden sowohl in stationären Geräten, wie etwa Anästhesie- oder Beatmungssystemen, sowie in sogenannten Kreislaufatemsystemen, die für Rettungseinsätze und zum Tauchen eingesetzt werden, verwendet. Wesentliches Merkmal der beschriebenen Atemluftversorgungen ist, dass die Atemluft zur Versorgung eines Menschen, insbesondere eines Patienten oder einer Rettungskraft, in einem geschlossenen Kreislauf geführt wird. Innerhalb dieses geschlossenen Kreislaufes muss der vom Menschen ausgeatmeten Luft zunächst ein Teil des in dieser enthaltenen CO₂ entfernt werden. Weiterhin wird der Luft oftmals Sauerstoff zugeführt und die Luft bedarfsgerecht temperiert.

Werden entsprechende Systeme für Anästhesiegeräte eingesetzt, so wird zunächst das vom Patienten ausgeatmete Atemgas von CO₂ befreit und anschließend mit Sauerstoff und volatilem Narkosemittel angereichert. In einem derartigen geschlossenen Verfahren wird die Ausnutzung von Sauerstoff und volatilen Narkosegasen erheblich verbessert und sowohl Kosten eingespart als auch die Umwelt geschont.

In den bekannten Atemkreislaufsystemen mit Rückatemsystem erfolgt das Entziehen von CO₂ aus dem Atemgas in der Regel mit sogenanntem Atemkalk. Bei der Absorption von CO₂ entsteht sowohl Wärme als auch Feuchte, da es sich hierbei um eine exotherme Reaktion handelt. Problematisch hierbei ist, dass die bei der CO₂-Absorption anfallende Feuchte in den nachgeschalteten Atemsystemkomponenten, insbesondere an Sensoren oder Ventilen, auskondensieren kann und so zu Funktionsbeeinträchtigungen oder sogar Ausfällen führen kann.

Um entsprechende Beeinträchtigungen zu vermeiden, sind etwa auf dem Gebiet der Anästhesiegeräte elektrische bzw. aktiv geregelte Heizsysteme bekannt, durch die ein Anfall von Feuchtigkeit im Atemsystem verhindert oder zumindest dafür gesorgt wird, dass eine entsprechende Kondensation an speziell hierfür vorgesehenen Stellen auftritt. Derartige Systeme verfügen oftmals über eine vergleichsweise aufwendige Regelungstechnik, sind teuer und haben einen erhöhten Energieverbrauch.

Aus der DE 10 2009 007 980 A1 ist ein Kohlendioxidabsorber für ein Atemkreislaufsystem bekannt, der an der Oberseite über einen Gaseinlass und einen Gasauslass und im unteren Bereich über einen Kondensatauffangbehälter verfügt. Auf einem Siebblech im Absorbergehäuse befindet sich eine körnige Schüttung aus Atemkalk, der während des Betriebs Kohlenstoffdioxid eines Patienten bindet. Das ausgeatmete Gas gelangt über den Gaseinlass und einen mittig im Absorbergehäuse angeordneten Kanal in ein Sammelvolumen unterhalb des Siebbleches und von dort über das Siebblech und den Atemkalk zum Gasauslass. Weiterhin befindet sich im unteren Bereich des Sammelvolumens ein Kondensatauffangbehälter mit einer Schicht aus einer wasserbindenden Substanz in Form eines superabsorbierenden Polymers, die das anfallende Kondensat auffängt und speichert. Die Substanz verhindert so ein Zurückfließen von Kondensat in den Atemkalk.

Eine alternative technische Lösung zur Entfernung von Feuchtigkeit aus der ausgeatmeten Luft im Beatmungskreis, die auf dem Einsatz eines Raumtemperatur-Wärmetauschers beruht, ist aus der DE 10 2006 040 886 A1 bekannt. In dem Beatmungskreis ist ein Wärmetauscher zur Entfernung von Wasserdampf aus den Beatmungsgasen zur Verhinderung der Kondensation innerhalb des Beatmungskreises vorgesehen. Der Wärmetauscher ist in diesem Fall stromabwärts zu einem CO₂-Absorber angeordnet und erhält die Beatmungsgase aus dem CO₂-Absorber, bevor sie zu dem Einatemzweig des Beatmungskreises strömen. Der Wärmetauscher enthält eine Anzahl von Einströmrohren und Ausströmrohren, die jeweils zu einem Sumpf offen sind, der an dem Wärmetauscher abnehmbar befestigt ist. In dem Sumpf sammelt sich das Kondensat, das aus den Beatmungsgasen innerhalb des Wärmetauschers kondensiert ist.

Des weiteren sind auch folgende Dokumente bekannt, EP 2 374 509, US 2002/0148471, WO 99/33523, DE 100 47 137, US 2010/0199993, US 2007/0051367.

Den bekannten Atemkreislaufsystemen ist gemein, dass der Anfall von Feuchte regelmäßig nicht verhindert wird, sondern die Kondensation im günstigsten Fall gezielt in einen bestimmten Bereich gelenkt wird, der in Folge dieser technischen Lösung vom Anwender gewartet und beachtet werden müssen.

Ausgehend von den bekannten Vorrichtungen zur Versorgung eines Menschen mit Atemluft, die über ein Rückatemsystem verfügen, liegt der Erfindung die Aufgabe zugrunde, diese Systeme derart weiterzubilden, dass Feuchte dort aufgefangen und gesammelt wird, wo sie entsteht und somit ein Transport von Feuchte in den Beatmungskreis zuverlässig vermindert wird. Hierbei ist es wünschenswert, dass sich Feuchte an einer Stelle sammelt, bei der es sich ohnehin um einen Wartungspunkt für den Anwender handelt. Auf bevorzugte Weise soll sich das aufgrund der exothermen Reaktion im CO₂-Absorber bildende Kondensat im Bereich des CO₂-Absorbers sammeln, da dieser, den Atemkalk enthaltende Absorber ohnehin regelmäßig gewartet werden muss. Da sich der Atemkalk nach einer gewissen Zeit aufgebraucht hat, muss dieser getauscht werden. Ein weiterer wesentlicher Teil der Aufgabe besteht darin, die bei der exothermen Reaktion im Kohlenstoffdioxidabsorber entstehende Wärme auf geeignete Weise aus der Reaktionszone abzuführen.

Die zuvor erläuterte Aufgabe wird mit einer Vorrichtung gemäß Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Erfindungsgemäß ist eine Vorrichtung zur Atemluftversorgung eines Menschen mit einem im Luftversorgungskreislauf angeordneten Rückatemsystem, das mittels eines CO₂-Absorbers wenigstens teilweise in der Ausatemluft des Menschen befindliches CO₂ entfernt und die Ausatemluft derart aufbereitet, dass die aufbereitete Luft dem Menschen wieder als Einatemluft zuführbar ist, und mit einem Kondensatauffangbehälter, in dem sich im Luftversorgungskreislauf bildendes Wasser auffangbar ist, derart weitergebildet worden, dass der Kondensatauffangbehälter wenigstens teilweise unterhalb einer Reaktionszone des. CO₂-Absorbers angeordnet ist und dass im CO₂-Absorber wenigstens ein Wärmeübertrager vorgesehen ist, durch den Wärme aus der den CO₂-Absorber durchströmenden, sich aufgrund der in der Reaktionszone des CO₂-Absorbers stattfindenden exothermen CO₂-Absorptionsreaktion erwärmenden Luft abgeführt wird. Grundsätzlich ist es hierbei denkbar, einen oder eine Mehrzahl von Wärmeübertragern vorzusehen, die sich wenigstens teilweise mittelbar oder unmittelbar mit der Reaktionszone in Kontakt finden und/oder die in einem der Reaktionszone strömungstechnisch nachgeschalteten Bereich angeordnet sind. Die erfindungsgemäße technische Lösung zeichnet sich somit dadurch aus, dass die sich innerhalb der Reaktionszone des CO₂-Absorbers bildende Feuchte direkt in einen sich unterhalb der Reaktionszone im Gehäuse des CO₂-Absorbers befindenden Kondensatauffangbehälter abgeleitet und gleichzeitig die sich innerhalb der Reaktionszone aufgrund der exothermen Reaktion bildende Wärme abgeführt wird. Durch diese Maßnahmen wird insbesondere sichergestellt, dass den CO₂-Absorber trockenere und kühlere Luft verlässt.

Gemäß einer ersten speziellen Ausführungsform der Erfindung ist der Wärmeübertrager in Form eines Wärmeleitbleches ausgeführt. Das Wärmleitblech ist entweder oberhalb des im CO₂-Absorber, vorzugsweise als Schüttung vorgesehenen Kalks oder aber zumindest teilweise innerhalb des Kalks angeordnet. Auf bevorzugte Weise ist ein derartiges Wärmeleitblech quer zur Gasströmungsrichtung angeordnet und verfügt über geeignete Durchbrüche, durch die das Atemgas strömen kann. Ein derartiges Wärmeleitelement kann beispielsweise in Form eines Lochbleches aus einem gut wärmeleitfähigen Material ausgeführt sein. Gemäß einer speziellen Ausführungsform der Erfindung sind an dem Wärmeleitelement, insbesondere dem Wärmeleitblech, Heizfahnen vorgesehen, die von dem Wärmeleitelement abstehen und in die Reaktionszone, insbesondere in die Atemkalk-Schüttung hineinragen. Auf diese Weise ist es möglich, eine Wärmeabführung innerhalb der Reaktionszone großflächig zu realisieren. Bei der Dimensionierung und Ausführung der Heizfahnen wird auf vorteilhafte Weise berücksichtigt, dass sich mit fortschreitender Betriebsdauer des CO₂-Absorbers in Abhängigkeit der Sättigung des Kalks mit Kohlenstoffdioxid die Reaktionszone und somit der Bereich, in dem aufgrund der exothermen Reaktion vorwiegend Feuchtigkeit und Wärme gebildet wird, in Strömungsrichtung, also bei einer Durchströmung von unten nach oben weiter nach oben, verlagert. Ausgehend von den zuerst angeströmten Bereichen des Kalks, insbesondere einer Kalkschüttung, verlagert sich die Reaktionszone daher mit zunehmender Betriebsdauer in die später angeströmten, in Strömungsrichtung weiter hinten liegenden Bereiche.

In Ergänzung zu einem ersten Wärmeübertrager, der hinter oder wenigstens bereichsweise innerhalb des Atemkalks angeordnet und bevorzugt in Form eines Wärmeleitelements ausgeführt ist, ist es denkbar, einen zusätzlichen Wärmeübertrager vorzusehen, der innerhalb des CO₂-Absorbers dem ersten Wärmeübertrager in Strömungsrichtung der Atemluft nachgeschaltet ist. In diesem Fall findet eine weitere Abkühlung des Atemgases statt nachdem dieses den ersten Wärmeübertrager bereits durch- oder umströmt hat und bevor das Atemgas den CO₂-Absorber, insbesondere das Gehäuse des Absorbers, verlassen hat.

Gemäß einer besonderen Weiterbildung der Erfindung ist zumindest ein Element zur Wärmeisolierung vorgesehen, das den ersten Wärmeübertrager und/oder die Wärmeleitelemente gegenüber ihrer Umgebung bzw. benachbarten Bauteilen wärmetechnisch isoliert. Insbesondere verfügt der erste Wärmeübertrager zumindest bereichsweise über eine Wärmeisolierung, die bevorzugt aus einem geeigneten Kunststoffmaterial ausgeführt ist. Eine derartige Wärmeisolierung ist vor allem in Fällen sinnvoll, in denen zusätzlich zum ersten Wärmeübertrager ein weiterer diesem nachgeschalteter Wärmeübertrager vorgesehen ist. Da der erste Wärmeübertrager auf geeignete Weise ein Wärmeleitblech aufweist, das zumindest in der Nähe des zusätzlichen Wärmeübertragers angeordnet ist, ist eine thermische Isolation in diesem Bereich, insbesondere zwischen dem ersten und dem zweiten Wärmeübertrager sinnvoll. Sofern eine Wärmeisolierung gleichzeitig einen vom Atemgas durchströmten Strömungskanal begrenzt ist es weiterhin sinnvoll, wenn die Wärmeisolierung gleichzeitig derart ausgeführt ist, dass eine Gasdichtigkeit gewährleistet ist. Dieses kann wiederum vorzugsweise durch Einsatz eines Kunststoffs, insbesondere eines Elastomers, wie etwa Silikon, TPE oder EPDM, realisiert werden.

In einer speziellen Ausführungsform ist das Gehäuse des CO₂-Absorbers zumindest abschnittsweise aus einem mindestens translucenten bzw. transparenten Kunststoff gefertigt. Dies hat den Vorteil, dass eine Verfärbung des Kalkes, die auftritt, wenn der Kalk erschöpft ist oder teilweise auch, wenn er ausgetrocknet ist und damit seine Funktion nicht mehr wahrnehmen kann, sichtbar ist. Dieser Farbumschlag ist ein wichtiges Signal für den Anwender eines Gerätes, dass der Atemkalk getauscht werden muss. In einer besonderen Ausführungsform steht der Kondensatbehälter im unmittelbaren Kontakt mit einem Behälter, in dem sich der Atemkalk befindet, so dass bei der Entnahme der Atemkalkpatrone gleichzeitig der Kondensatbehälter entfernt wird. Selbstverständlich ist es ebenfalls denkbar, den Kondensatauffangbehälter als separates Bauteil auszuführen, das entleert wird, sobald die Atemkalkpatrone getauscht werden muss. In jedem Fall ist es vorteilhaft, wenn der Kondensatauffangbehälter derart dimensioniert ist, dass er frühestens entleert werden muss, wenn sich auch der Atemkalk innerhalb des CO₂-Absorbers verbraucht hat. Auf vorteilhafte Weise kann hierfür innerhalb des Kondensatauffangbehälters ein spezielles Absorbermaterial vorgesehen sein, das die auskondensierende Feuchtigkeit aus dem Atemgas aufnimmt.

Auf vorteilhafte Weise wird die aus dem CO₂-Absorber abgeführte Wärme innerhalb des Beatmungskreislaufes genutzt, um beispielsweise gezielt Sensoren und/oder Ventile zu beheizen. Auf diese Weise kann die ohnehin anfallende Wärme genutzt werden, um an den zuvor beschriebenen Komponenten zuverlässig ein Auskondensieren von Feuchtigkeit zu verhindern. Besonders bevorzugt wird die mithilfe des erfindungsgemäß vorgesehenen ersten und/oder zusätzlichen Wärmeübertragers abgeführte Wärme für einen Strömungssensor und/oder einen Drucksensor innerhalb des Atemkreislaufes verwendet. Neben der Reduzierung des CO₂-Anteils im Atemgas findet beim erfindungsgemäß ausgeführten Atemkreislaufsystem mit Rückatmung eine weitere Aufbereitung der Atemluft statt, wobei dem Atemgas Sauerstoff, wenigstens ein Narkosemittel und/oder Schmerzmittel zugesetzt wird. Die erfindungsgemäß ausgeführte Vorrichtung eignet sich somit insbesondere zum Einsatz im Zusammenhang mit Anästhesiegeräten, Beatmungsgeräten und/oder Kreislaufatemgeräten zum Tauchen oder für Rettungseinsätze.

Erfindungswesentlich ist jeweils, dass in den entsprechenden Systemen die innerhalb des Kohlenstoffdioxid-Absorbers anfallende Wärme abgeführt und vorzugsweise für weitere Komponenten innerhalb des Kreislaufatemsystems genutzt wird. Hierbei wird die Abkühlung der Atemluft innerhalb des CO₂-Absorbers derart durchgeführt, dass die sich hierbei bildende Feuchtigkeit innerhalb des CO₂-Absorbergehäuses verbleibt und in einen speziell hierfür vorgesehenen Kondensatauffangbehälter bzw. Kondensatauffangbereich des CO₂-Absorbergehäuses gelangt.

Im Folgenden wird die Erfindung ohne Beschränkung des allgemeinen Erfindungsgedankens unter Bezugnahme auf die Figuren anhand von Ausführungsbeispielen erläutert. Dabei zeigen:

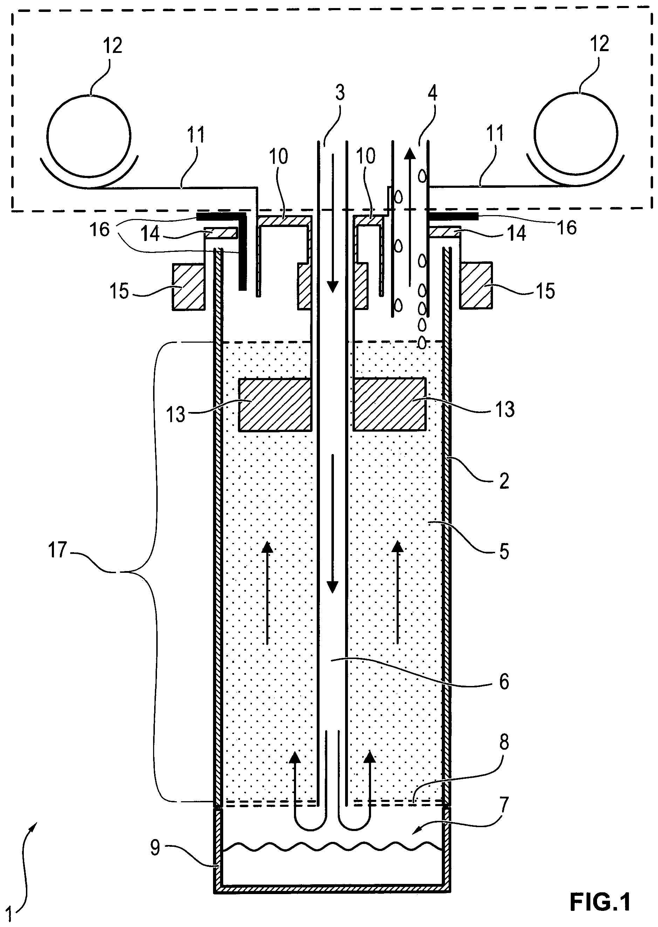

- Figur 1:: Schnittansicht eines erfindungsgemäß ausgeführten Kohlendioxid-Absorbers mit Atemkalkschüttung.

Figur 1 zeigt eine erfindungsgemäß ausgeführte Absorbereinheit 1 zur Absorption von Kohlenstoffdioxid (CO₂), wie sie auf geeignete Weise in Kreislaufatemgeräten mit Rückatemsystem einsetzbar ist. Das Gehäuse 2 der Absorbereinheit 1 verfügt über einen Gaseinlass 3, über das vom Menschen abgegebenes exspiratorisches Atemgas dem Absorber 1 zugeführt wird. Ferner ist ein Gasauslass 4 vorgesehen, durch den Atemgas, dem Kohlenstoffdioxid entzogen wurde, den Absorber 1 verlässt.

Auf einem Siebblech 8 in der Absorbereinheit 1 befindet sich eine Atemkalkschüttung 5, durch die vom Menschen ausgeatmetes Atemgas geleitet wird, um dieses zumindest teilweise von Kohlenstoffdioxid zu befreien. Das ausgeatmete Atemgas gelangt vom Gaseinlass 3 über einen mittig im Absorbergehäuse 2 angeordneten Kanal 6 in ein Sammelvolumen 7 unterhalb des Siebbleches 8 und von dort über das Siebblech 8 und den Atemkalk 5 schließlich zum Gasauslass 4.

Im unteren Bereich des Sammelvolumens 7 der Absorbereinheit 1, also ebenfalls unterhalb des Siebbleches 8 mit der darauf gelagerten Atemkalkschüttung 5, in der sich die jeweilige CO₂-Reaktionszone 17befindet, ist ein Kondensatauffangbehälter 9 vorgesehen. In diesem Kondensatauffangbehälter 9 sammelt sich das im Absorber 1 anfallende Kondensat bzw. die sich bildende Feuchtigkeit. Das Kondensat bildet sich innerhalb der Absorbereinheit 1 einerseits aufgrund der im Atemkalk 5 stattfindenden Reaktion, bei der Wasser freigesetzt wird sowie andererseits aufgrund der gezielten Abkühlung der Atemluft, die unmittelbar einen Anstieg der relativen Feuchte der Luft zur Folge hat. Das vom Menschen ausgeatmete Atemgas durchströmt somit den in der Absorbereinheit 1 angeordneten Atemkalk 5 von unten nach oben und strömt schließlich als von CO₂ gereinigtes Atemgas über den Gasauslass 4 wieder zurück in das Atemsystem.

Als Atemkalk 5 wird eine granulatartige Mischung aus Calciumhydroxid (Ca(OH)₂) und Natriumhydroxid (NaOH) verwendet. Während das vom Menschen ausgeatmete Atemgas die Atemkalkschüttung 5 der Absorbereinheit 1 durchströmt finden folgende chemische Reaktionen statt:

CO₂ + H₂O ↔ H₂CO3

H₂CO₃ + 2 NaOH ↔ Na₂CO₃ + 2 H₂O

Na₂CO₃ + Ca(OH)₂ - ↔ CaCO₃ + 2 NaOH

Aufgrund der in der Reaktionszone 17 stattfindenden Reaktionen entsteht Wärme und Wasser. Durchschnittlich ausgeführte Atemkalke 5 sind in der Lage 10 - 15 Liter CO₂ je 100 g Schüttgut zu absorbieren. Weiterhin ist dem Atemkalk 5 ein pH-Indikator beigemischt, der bei niedrigem pH-Wert seine Farbe von weiß nach violett ändert und damit anzeigt, dass der Atemkalk 5 verbraucht ist.

Sofern der Atemkalk 5 noch unverbraucht ist, reagiert das CO₂ im exspiratorischen Atemgas bereits direkt an der Eintrittsstelle im Kalk 5, so dass dieser Bereich anfänglich die Reaktionszone 17 bildet. Bei der Reaktion bzw. der Absorption von CO₂ entsteht aufgrund der hier stattfindenden exothermen Reaktion sowohl Wärme als auch Feuchte. Mit zunehmendem Verbrauch des Kalkes 5 wandert die Reaktionszone 17 innerhalb des CO₂-Absorbers 1 weiter nach oben. Je weiter die Reaktionszone 17 nach oben wandert, umso näher rückt auch die Zone, in der sich Feuchtigkeit bildet, in Richtung des Gasauslasses 4 der CO₂-Absorbereinheit 1 und somit dem Atemkreislauf. Hierbei besteht grundsätzlich die Gefahr, dass warme und feuchte Luft in den Atemkreislauf gelangt, in dem sie dann auskondensiert und insbesondere bei Kondensation im Bereich von Funktionselementen, wie etwa Ventilen oder Druck- und Strömungssensoren, für deren Ausfall verantwortlich sein kann.

Um diesen Effekt zuverlässig zu vermeiden, ist innerhalb des Gehäuses 2 der CO₂-Absorbereinheit 1 ein Wärmeübertrager 10 vorgesehen, der die sich in der Reaktionszone 17 bildende Wärme aufnimmt, die daraufhin aus der Reaktionszone 17 , insbesondere auch aus dem CO₂-Absorbergehäuse 2, abgeführt wird. Die so abgeführte Wärme wird mittels geeigneter Wärmeleitelemente 11 an relevante Bauteile 12, die wenigstens bereichsweise mit dem Atemgas in Berührung stehen, insbesondere an Druck- und Strömungssensoren innerhalb des Atemkreislaufsystems, weitergeleitet. Im Bereich dieser Bauteile 12 kommt es zu einer gezielten Erwärmung, so dass Kondensationserscheinungen in diesem Bereich vermieden werden. Auf diese Weise halten die entsprechenden Bauteile 12 ein Temperaturniveau, das über der Umgebungstemperatur liegt und so eine Kondensationsneigung herabgesenkt wird.

Je höher im Verlauf des Atemkalkverbrauches die Reaktionszone 17 innerhalb der Atemkalkschüttung 5 wandert, umso mehr Wärme wird von dem in Form einer Wärmeleitplatte ausgebildeten Wärmeübertrager 10 aufgenommen. Um die Wärmeabführung aus der Reaktionszone 17 weiter zu verbessern, sind spezielle Wärmeleitfahnen 13 innerhalb der Atemkalkschüttung 5 vorgesehen, die in thermischem Kontakt mit dem Wärmeübertrager 10 stehen. Derartige Wärmeleitfahnen 13 können generell sowohl innerhalb der Atemkalkschüttung 5 als auch in einem der Schüttung 5 nachgeschalteten Bereich im Absorbergehäuse 2 angeordnet sein.

Gemäß dem in Figur 1 dargestellten Ausführungsbeispiel besteht eine weitere Maßnahme zur Kondensationsvermeidung in der Abkühlung des aus der Schüttung 5 austretenden, gereinigten Atemgases. Hierbei wird der gereinigte Gasstrom durch einen weiteren Wärmeübertrager 14 geleitet, der durch Umgebungsluft und somit auf Raumtemperatur gekühlt ist. Ein entsprechender weiterer Temperaturaustausch kann ebenfalls durch geeignet vorgesehene Kühlelemente 15 oder auch durch einen gelenkten Kühlluftstrom unterstützt werden. In diesem Zusammenhang ist es denkbar, dass ein derartiger Kühlluftstrom mit Hilfe einer Ventilatoreinheit eines angeschlossenen Anästhesie-, Beatmungs- oder Kreislaufatemgerätes in Richtung der CO2-Absorbereinheit 1 ausgeblasen oder aus dieser abgesaugt wird.

Sofern wie in Figur 1 gezeigt innerhalb des Kohlenstoffdioxid-Absorbers 1 zwei Wärmeübertrager 10, 14 vorgesehen sind, sind diese mithilfe einer Wärmeisolierung 16 thermisch voneinander isoliert. Eine derartige Wärmeisolierung 16 kann beispielsweise durch Aufbringen einer Kunststoffschicht, insbesondere eines Elastomers, begünstigt werden.

Aufgrund der durch den zweiten Wärmeübertrager 14 bewirkten weiteren Abkühlung des Atemgases, bildet sich zumindest zeitweise auch im Bereich dieses Wärmeübertragers 14 Kondensat, das in den Bereich des Kondensatauffangbehälters abgeleitet werden muss. Das Kondensat strömt hierbei dem Atemgas entgegen durch den Kalk und gelangt schließlich in den Kondensatauffangbehälter 9 im unteren Bereich des CO₂-Absorbergehäuses 2. Da das vom zweiten Wärmeübertrager 14 in Richtung des Kondensatauffangbehälters 9 herabströmende Kondensat den ersten Wärmeübertrager 10 passieren muss, wird es derart gelenkt, dass es am ersten Wärmeübertrager 10 nicht direkt entlangläuft, um so ungewünschte Abkühlung zu verhindern. Insbesondere sind die thermisch mit dem ersten Wärmeübertrager 10 verbundenen Wärmeleitfahnen 13 nicht unterhalb der Flächen des zweiten Wärmeübertragers 14 angeordnet, an denen sich Kondensat bildet und von denen es schließlich herabfließt.

Im Kondensatauffangbehälter 9 bzw. Kondensatreservoir wird das Kondensat beim zyklischen Kalkaustausch automatisch mit entsorgt. Aus diesem Grund ist das Füllvolumen des Kondensatauffangbehälters 9 bevorzugt derart dimensioniert, dass es innerhalb eines normal üblichen Tauschzyklus nicht überläuft. Der Füllstand des Kondensatauffangbehälters 9 ist von außen sichtbar, damit vom Anwender rechtzeitig eine Überfüllung erkannt und entsprechend reagiert werden kann.

Um eine optische Kontrolle der Atemkalkschüttung 5 zu erleichtern, ist das CO₂-Absörbergehäuse 2 wenigstens abschnittsweise aus einem transluzenten oder transparenten Kunststoff gefertigt. Auf diese Weise wird sichergestellt, dass der Anwender eine Verfärbung des Kalkes 5 zuverlässig feststellen kann. Eine Verfärbung des Kalkes 5 findet statt, sobald dieser erschöpft ist oder teilweise auch, sobald er ausgetrocknet ist. Eine Verfärbung findet somit in Fällen statt, in denen der Atemkalk 5 seine eigentliche Funktion nicht mehr ausführen kann. Der Farbumschlag stellt somit ein wichtiges Signal für den Anwender dar, so dass dieser zuverlässig erkennen kann, dass der Atemkalk getauscht werden muss.

Die entsprechende Absorbereinheit 1, insbesondere deren Atemkalkschüttung 5, ist derart ausgeführt, dass ein maximaler oberer Kalkfüllstand nicht überschritten wird, damit dem Anwender in jedem Fall die obere maximale Füllmarke erkennbar bleibt. Der zweite Wärmeübertrager 14 innerhalb des CO₂-Absorbergehäuses 2 ist oberhalb der Atemkalkschüttung 5 angeordnet, so dass,die Sicht auf den Atemkalk 5 für den Anwender nicht erschwert wird. Sowohl der erste Wärmeübertrager 10 in Form einer Wärmeleitplatte wie auch der zweite, raumluftgekühlte Wärmeübertrager 14 werden aus Aluminium, Kupfer, Messing oder aus einem wärmeleitenden Kunststoff hergestellt. Sofern sich die beiden Wärmeübertrager 10, 14 in unmittelbarer Nähe befinden, ist zwischen diesen eine Wärmeisolierung vorgesehen, die vorzugsweise auch gasdicht ausgeführt ist. Diese Wärmeisolierung kann wiederum ein Kunststoff- bzw. Elastomermaterial, insbesondere Silikon, TPE oder EPDM, enthalten. Für die Herstellung eines derartigen Wärmeübertragers ist es vorteilhaft, diesen mithilfe eines werkzeuggebundenen Zwei-Komponenten-Spritzverfahrens aus einer Kombination aus wärmeleitfähigen Kunststoffen und isolierenden und dichtenden Elastomeren herzustellen.

Die erfindungsgemäße technische Lösung, die darauf beruht, dass die Abkühlung und die Auskondensation von Feuchtigkeit gezielt an einem Ort herbeigeführt werden, zeichnet sich durch einen einfachen Aufbau aus und gewährleistet dennoch eine hohe Ausfallsicherheit. Hierbei ist es besonders vorteilhaft, dass die Feuchtigkeit in einem Bereich ausfällt, in dem ohnehin ein Wartungspunkt eines Atemluftversorgungssystems liegt. Wesentlich hierbei ist, dass ein Anwender ohnehin daran gewöhnt ist, diesen Bereich durch eine visuelle Kontrolle zu überprüfen und turnusgemäß die Atemkalkpatrone zu wechseln. Durch die Kopplung mit einer aktiven elektrischen Heizung kann eine noch größere Sicherheit in der Kondensationsvermeidung bei Systemen zur Atemluftversorgung von Menschen, insbesondere von Patienten oder von Rettungskräften, erzielt werden.

### Bezugszeichenliste

- 1: CO₂-Absorbereinheit
- 2: Absorbergehäuse
- 3: Gaseinlass
- 4: Gasauslass
- 5: Kalkschüttung
- 6: Gaskanal
- 7: Sammelvolumen
- 8: Siebblech
- 9: Kondensatauffangbehälter
- 10: erster Wärmeübertrager
- 11: Wärmeleitelement
- 12: Funktionsbauelement
- 13: Wärmeleitfahne
- 14: zweiter Wärmeübertrager
- 15: Kühlelement
- 16: Wärmeisolierung
- 17: Reaktionszone

## Patentansprüche

1. Vorrichtung zur Atemluftversorgung eines Menschen mit einem im Luftversorgungskreislauf angeordneten Rückatemsystem, das mittels eines mit Kalk gefüllten CO₂-Absorbers (1) wenigstens teilweise in der Ausatemluft des Menschen befindliches CO₂ entfernt und die Ausatemluft derart aufbereitet, dass die aufbereitete Luft dem Menschen wieder als Einatemluft zuführbar ist, und mit einem Kondensatauffangbehälter (9), in dem sich im Luftversorgungskreislauf bildendes Wasser auffangbar ist, wobei der Kondensatauffangbehälter (9) wenigstens teilweise unterhalb einer Reaktionszone (17) des CO₂-Absorbers (1) angeordnet ist und im CO₂-Absorber (1) wenigstens ein Wärmeübertrager (10, 14) vorgesehen ist, durch den Wärme aus der den CO₂-Absorber (1) durchströmenden, sich aufgrund der in der Reaktionszone des CO₂-Absorbers (1) stattfindenden exothermen CO₂-Absorptionsreaktion erwärmenden Luft abgeführt wird, **dadurch gekennzeichnet, dass** die Vorrichtung ein Wärmeleitelement (11) aufweist und dass die von dem wenigstens einen Wärmeübertrager (10, 14) aus der Luft abgeführte Wärme über dieses Wärmeleitelement (11) zu wenigstens einem zumindest bereichsweise innerhalb des Luftversorgungskreislaufes angeordneten Funktionsbauteil (12) leitbar ist.

2. Vorrichtung nach Anspruch 1,
dass wenigstens ein Wärmeübertrager (10, 14) in Strömungsrichtung der Luft hinter der Reaktionszone (17) im CO₂-Absorber (1) angeordnet ist.

3. Vorrichtung nach Anspruch 1 oder 2,
**dadurch gekennzeichnet, dass** wenigstens ein Wärmeübertrager (10, 14) zumindest bereichsweise innerhalb der Reaktionszone (17) angeordnet ist.

4. Vorrichtung nach einem der Ansprüche 1 bis 3,
**dadurch gekennzeichnet, dass** zumindest ein Wärmeübertrager (10, 14) als plattenförmiges Wärmeleitelement ausgeführt ist.

5. Vorrichtung nach einem der Ansprüche 1 bis 4,
**dadurch gekennzeichnet, dass** zumindest ein Wärmeübertrager (10, 14) an seiner Außenfläche wenigstens abschnittsweise eine kunststoffhaltige Wärmeisolierung (16) aufweist.

6. Vorrichtung nach einem der Ansprüche 1 bis 5,
**dadurch gekennzeichnet, dass** wenigstens ein Wärmeübertrager (10, 14) zumindest eine in die Reaktionszone (17) reichende Wärmeleitfahne aufweist.

7. Vorrichtung nach einem der Ansprüche 1 bis 6,
**dadurch gekennzeichnet, dass** wenigstens ein Wärmeübertrager (10, 14) mit Umgebungsluft kühlbar ist.

8. Vorrichtung nach einem der Ansprüche 1 bis 7 ,
**dadurch gekennzeichnet, dass** das Funktionsbauteil (12) als Strömungssensor und/oder als Drucksensor ausgeführt ist.

9. Vorrichtung nach einem der Ansprüche 1 bis 8,
**dadurch gekennzeichnet, dass** im Kondensatauffangbehälter (9) wenigstens ein Material, das Wasser bindet, angeordnet ist.

10. Vorrichtung nach einem der Ansprüche 1 bis 9,
**dadurch gekennzeichnet, dass** der Luft während der Aufbereitung Sauerstoff und/oder ein Narkosegas zugesetzt wird.

11. Anästhesiegerät, Beatmungsgerät und/oder Kreislaufatemgerät zum Tauchen oder für Rettungseinsätze mit einer Vorrichtung nach einem der Ansprüche 1 bis 10.

## Claims

1. Device for supplying breathing air to a person, with a rebreathing system which is arranged in the air supply circuit and which, by means of a chalk-filled CO₂ absorber (1), removes at least some of the CO₂ present in the air exhaled by the person and processes the exhaled air in such a way that the processed air can be fed back to the person as air for inhalation, and with a condensate collection tank (9) in which water forming in the air supply circuit can be collected, wherein the condensate collection tank (9) is arranged at least partly under a reaction zone (17) of the CO₂ absorber (1), and at least one heat exchanger (10, 14) is provided in the CO₂ absorber (1) and removes heat from the air which flows through the CO₂ absorber (1) and which heats on account of the exothermic CO₂ absorption reaction taking place in the reaction zone of the CO₂ absorber (1), **characterized in that** the device has a heat-conducting element (11), and **in that** the heat removed from the air by the at least one heat exchanger (10, 14) can be conducted via this heat-conducting element (11) to at least one functional component (12), which is arranged at least in part inside the air supply circuit.

2. Device according to Claim 1, that at least one heat exchanger (10, 14), seen in the direction of flow of the air, is arranged downstream from the reaction zone (17) in the CO₂ absorber (1).

3. Device according to Claim 1 or 2, **characterized in that** at least one heat exchanger (10, 14) is arranged at least in part inside the reaction zone (17).

4. Device according to one of Claims 1 to 3, **characterized in that** at least one heat exchanger (10, 14) is configured as a plate-shaped heat-conducting element.

5. Device according to one of Claims 1 to 4, **characterized in that** at least one heat exchanger (10, 14) has, on at least parts of its outer surface, a plastic-containing heat insulation (16).

6. Device according to one of Claims 1 to 5, **characterized in that** at least one heat exchanger (10, 14) has at least one heat-conducting tab reaching into the reaction zone (17).

7. Device according to one of Claims 1 to 6, **characterized in that** at least one heat exchanger (10, 14) can be cooled with ambient air.

8. Device according to one of Claims 1 to 7, **characterized in that** the functional component (12) is configured as a flow sensor and/or as a pressure sensor.

9. Device according to one of Claims 1 to 8, **characterized in that** at least one material that binds water is arranged in the condensate collection tank (9).

10. Device according to one of Claims 1 to 9, **characterized in that** oxygen and/or an anaesthetic gas is added to the air during the processing.

11. Anaesthesia apparatus, ventilation apparatus and/or rebreather apparatus for diving or for rescue applications, with a device according to one of Claims 1 to 10.

## Revendications

1. Dispositif pour l'alimentation en air respiratoire d'une personne, équipé d'un système de réinspiration disposé dans le circuit d'alimentation en air, qui élimine au moins en partie le CO₂ présent dans l'air d'expiration de la personne au moyen d'un absorbeur de CO₂ (1) rempli de chaux et prépare l'air d'expiration de telle manière que l'air préparé puisse être de nouveau fourni à la personne en tant qu'air d'inspiration, et d'un récipient de collecte de condensat (9), dans lequel l'eau se formant dans le circuit d'alimentation en air peut être collectée, dans lequel le récipient de collecte de condensat (9) est disposé au moins en partie en dessous d'une zone de réaction (17) de l'absorbeur de CO₂ (1) et il est prévu dans l'absorbeur de CO₂ (1) au moins un échangeur de chaleur (10, 14), par lequel de la chaleur est évacuée hors de l'air traversant l'absorbeur de CO₂ et s'échauffant à cause de la réaction exothermique d'absorption de CO₂ qui se produit dans la zone de réaction de l'absorbeur de CO₂ (1),
**caractérisé en ce que** le dispositif présente un déflecteur de chaleur (11) et **en ce que** la chaleur évacuée hors de l'air par ledit au moins un échangeur de chaleur (10, 14) peut être conduite par ce déflecteur de chaleur (11) vers un composant fonctionnel (12) disposé au moins partiellement à l'intérieur du circuit d'alimentation en air.

2. Dispositif selon la revendication 1, **caractérisé en ce qu'**au moins un échangeur de chaleur (10, 14) est disposé dans l'absorbeur de CO₂ (1) après la zone de réaction (17) dans la direction d'écoulement de l'air.

3. Dispositif selon une revendication 1 ou 2, **caractérisé en ce qu'**au moins un échangeur de chaleur (10, 14) est disposé au moins partiellement à l'intérieur de la zone de réaction (17).

4. Dispositif selon l'une quelconque des revendications 1 à 3, **caractérisé en ce qu'**au moins un échangeur de chaleur (10, 14) est formé par un déflecteur de chaleur en forme de plaque.

5. Dispositif selon l'une quelconque des revendications 1 à 4, **caractérisé en ce qu'**au moins un échangeur de chaleur (10, 14) présente sur sa surface extérieure au moins partiellement une isolation thermique contenant une matière plastique (16).

6. Dispositif selon l'une quelconque des revendications 1 à 5, **caractérisé en ce qu'**au moins un échangeur de chaleur (10, 14) présente au moins une languette de guidage de chaleur pénétrant dans la zone de réaction (17).

7. Dispositif selon l'une quelconque des revendications 1 à 6, **caractérisé en ce qu'**au moins un échangeur de chaleur (10, 14) peut être refroidi avec l'air ambiant.

8. Dispositif selon l'une quelconque des revendications 1 à 7, **caractérisé en ce que** le composant fonctionnel (12) est formé par un détecteur d'écoulement et/ou par un capteur de pression.

9. Dispositif selon l'une quelconque des revendications 1 à 8, **caractérisé en ce qu'**au moins un matériau, qui lie l'eau, est disposé dans le récipient de collecte de condensat (9).

10. Dispositif selon l'une quelconque des revendications 1 à 9, **caractérisé en ce que** l'on ajoute de l'oxygène et/ou un gaz anesthésiant à l'air pendant la préparation.

11. Appareil d'anesthésie, appareil respiratoire et/ou appareil respiratoire autonome pour la plongée ou pour des opérations de sauvetage équipé d'un dispositif selon l'une quelconque des revendications 1 à 10.
